Europäisches Patentamt

European Patent Office

Office européen des brevets

(19)

(11) Numéro de publication : **0 467 795 A2**

(12) # DEMANDE DE BREVET EUROPEEN

(21) Numéro de dépôt : **91420223.9**

(51) Int. Cl.[5] : **A61K 31/07**

(22) Date de dépôt : **04.07.91**

(30) Priorité : **04.07.90 FR 9008781**

(43) Date de publication de la demande :
**22.01.92 Bulletin 92/04**

(84) Etats contractants désignés :
**AT BE CH DE DK ES FR GB GR IT LI LU NL SE**

(71) Demandeur : **PATRINOVE**
**39, rue du Lieutenant-Colonel-Prévost**
**F-69006 Lyon (FR)**
Demandeur : **TEXINFINE**
**60, rue Duguesclin**
**F-69006 Lyon (FR)**

(72) Inventeur : **Gutierrez, Gilles**
**39 Rue Lieutenant Colonel Prévost**
**F-69006 Lyon (FR)**

(74) Mandataire : **Monnier, Guy et al**
**Cabinet Monnier 150 Cours Lafayette B.P.**
**3058**
**F-69393 Lyon Cédex 03 (FR)**

(54) **Composition dermatologique ou cosmétique, contenant des dérivés de la vitamine A.**

(57) Application de vecteurs liposomiques paucilamellaires, à l'extérieur desquels sont fixées des molécules hydrophiles, au transport, à travers la membrane cellulaire, de molécules actives exprimant une affinité pour les transporteurs cytoplasmiques.

Ces molécules actives sont choisies parmi les molécules qui présentent une affinité pour les récepteurs ou les transporteurs de la vitamine A et des stéroïdes et donc une certaine analogie de structure avec ceux-ci et parmi les molécules qui manifestent un blocage de la formation des mélanosomes par détournement de l'activité enzymatique se traduisant par une hyperactivation des enzymes de dégradation des mélanines.

L'invention concerne également les compositions dermatologiques ou cosmétiques, à base de ces vecteurs et leurs applications à la supression ou à l'atténuation de dischromies ainsi qu' au traitement des mélanomes engendres par l'exposition solaire.

EP 0 467 795 A2

La présente invention concerne l'application de certains vecteurs liposomiques au transport, à travers la membrane cellulaire, de molécules actives, ainsi que l'utilisation de ces vecteurs notamment en dermatologie et cosmétique.

Les liposomes sont maintenant bien connus et ont été décrits et étudiés dans quelques ouvrages ou publications de référence auxquels on se rapportera utilement et parmi lesquels on peut citer :

– F. Puisieux et J. Delattre - LES LIPOSOMES, APPLICATIONS THERAPEUTIQUES - Techniques et Documentation.

– P. Machu et L. Leserman, LES LIPOSOMES EN BIOLOGIE CELLULAIRE ET PHARMACOLOGIE - Editions INSERM

G. Gregoriadis, Ed. Gregoriadis - LIPOSOMES AS DRUG CARRIERS - Wiley.

Les matières premières nécessaires à la préparation ou à la synthèse de ces liposomes sont essentiellement les suivantes :

a) un compose amphiphile, constitutif de la paroi du liposome, tel que :- les phospholipides, comme la lécithine, les phosphatidylcholines naturelles ou synthétiques, les sphingomyélines, les phosphatidylinositols, les phosphatidylsérines, les lysophosphatidylcholines, les dicétylphosphates.

– les tensio-actifs de synthèse ou naturels.

b) Eventuellement un composé de rigidification de la paroi du liposome, tel que

– un composé polycyclique comportant une chaîne ramifiée tel qu'un styrol, comme le cholestérol, les phytostérols, la fluorescéine acide et ses esters.

– certaines amines aromatiques

– certains sucres comme les polyoses, la streptromycine.

c) Eventuellement un compose conférant une charge positive ou négative à la surface de la membrane des liposomes, tel que le dipalmitoyl phosphatidylglycérol, pour obtenir une charge négative.

d) Eventuellement un agent antioxydant du compose amphiphile et / ou du composé de rigidification, tel que le DL-tocophérol.

e) La substance à encapsuler peut être solubilisée en milieu aqueux ou dans le composé amphiphile ou dans un milieu organique approprié.

Ces différentes matières sont solubilisées ou mises en suspension dans différents milieux aqueux et / ou organiques, afin de préparer les liposomes selon différentes méthodes ou voies d'obtention faisant intervenir ces milieux.

Sans entrer dans les détails et le classement des différentes méthodes de préparation, on peut dire que la plupart de celles-ci opèrent par étapes.

La première étape consiste à former une pseudo-membrane cellulaire et à transformer cette membrane en une vésicule stable. La deuxième étape consiste à améliorer les liposomes obtenus selon la première étape en les triant ou les sélectionnant par différents moyens physiques, tels que passage dans une presse de FRENCH, un filtre, une sonication etc... Cette étape permet par exemple de transformer des populations plurimodales de liposomes de différentes tailles en des liposomes de petites tailles unilamellaires.

La troisième et dernière étape consiste à éliminer les matières premières non utilisées dans le processus d'encapsulation, ainsi que le ou les milieux de solubilisation ou suspension par exemple par centrifugation, chromatographie.

Plus récemment, dans la demande de brevet français 89 09 2675 du 5 Juillet 1989 on a proposé une autre technique de production des liposomes par soumission d'une veine liquide à une alternance de surpressions et de dépressions, provoquant ainsi des phenomènes de cavitation .

On sait que par ailleurs que l'épiderme humain est colore par un pigment particulier : la mélanine, synthétisée par le mélanocyte.

Cette cellule est associée aux kératinocytes qui constituent le revêtement épidermique et qui sont des accepteurs du pigment. L'ensemble du mélanocyte et des kératinocytes qui leur sont associés constitue une unité fonctionnelle apelée unité pidermique de Mélanisation.

La mélanine joue un rôle important dans la protection contre les radiations solaires mais la coloration plus ou moins foncée qu'elle communique à la peau peut être ressentie comme un desagrément esthétique que l'on a autrefois souvent cherché à camoufler. Par ailleurs, les taches mélaniques cutanées qui s'accentuent ou apparaissent avec l'âge sont toujours mal supportées.

La mélanine est formée à partir de la tyrosine après une double oxydation contrôlée par les tyrosinases ; on aboutit alors à la dopaquinone, clef de voûte du processus métabolique conduisant aux deux groupes de pigments mélaniques, les eumélanines et les phaeomélanines.

On a proposé de nombreux produits destines à attenuer ou à effacer cette hyperpigmentation, mais leur utilisation n'est pas toujours sans danger.

Les dépigmentants classiques, agissent sur les deux premières phases de la transformation de la tyrosine, en empêchant son oxydation,selon des mécanismes mal connus.

Les sels de mercure,qui agissent en inhibant par compétition l'action du cuivre indispensable pour déclencher l'activité de la tyrosine, sont à proscrire pour des raisons évidentes de sécurité.

Les dérivés phénoliques, tels que l'hydroquinone et plus spécialement ses éthers, qui inhiberaient la synthèse de la tyrosinase, provoquent ainsi l'arrêt de la production de la mélanine.

Leur mauvaise tolérance les a fait exclure du champ d'application cosmétique.

Par ailleurs, la concentration nécessaire efficace est de 5 % et la limite officielle prévue pour la seule hydroquinone n'est que de 2 % rendant aléatoire l'effet dépigmentant.

Les substances réductrices comme la vitamine C empêcheraient la formation de dopaquinine par oxydation mais leur action dépigmentante est excessivement légère.

Quant aux corticoïdes qui agissent directement sur les mélanocytes, leur action est à proscrire pour des raisons de sécurité.

Enfin on rencontre des compositions dermatologiques ou cosmétiques renfermant, comme principe actif, de la vitamine A : après pénétration dans la cellule, cette vitamine A se fixe sur ses récepteurs ; le complexe forme active spécifiquement la réaction de fixation des corticoïdes, diminuant ainsi la teneur en ACTH qui est elle-mème un activateur de la MSGH ; la vitamine A traite alors les anomalies pigmentaires.La combinaison de ces deux effets est particulièrement mise en évidence dans le FR-C 2 591 105 qui, dans les exemples 11 et 12, décrit la préparation de compositions dermatologiques ou cosmétiques renfermant de la trétinoine (vitamine A acide) et de l'hydroquinone, compositions utilisées en application sur les taches pigmentaires de la peau.

De très récents travaux de M. Petkovich, N.J. Brand, A. Krust et P. Chambon, (NATURE, (1987) 330, 444 - 450), et V. Giguerre, Es. Ong, P. Segui et R.M. Evans, (NATURE (1987) 330, 624 - 629) ont demontré l'existence de récepteurs intracellulaires spécifiques de la vitamine A et de l'acide rétinoïque, en réalisant le clonage du DNA complémentaire, synthetisé à partir du RNA messager, porteur du code de la synthèse d'une protéine qui appartiendrait à la classe des facteurs inductifs de régulation et dont le mécanisme d'action serait semblable à celui des récepteurs hormonaux stéroïdiens.

Le mécanisme d'action original de ces substances peut être mis à profit dans la dépigmentation. Ces substances sont des analogues structuraux du rétinol.

Elles possèdent, comme lui, une activité pour les protéines transporteuses des stéroïdes qui jouent un rôle important dans la pigmentation.

L'activité dépigmentante de ces substances est basée sur l'hypothèse suivante : si on leur permet de parvenir au niveau cytoplasmique du mélanocyte, elles vont saturer la protéine transporteuse et donc empêcher la vitamine A d'exprimer son activité.

La fixation des molécules dudit principe actif avec ces récepteurs ou transporteurs d'une part et l'inhibition de l'activité régulatrice du transporteur d'autre part peuvent donc être interprétées par analogie avec l'interaction connue entre les anti-vitamines K, dérivés de la vitamine K et la vitamine K elle-mème : par exemple le bis-coumacétate d'éthyl vis-à-vis de la vitamine K.

La molécule du principe actif peut pénétrer dans les mélanocytes et se fixer sur des récepteurs spécifiques nucléaires. Le complexe forme n'active pas la production de mélanine. Le blocage des récepteurs de la vitamine A et des stéroïdes par un analogue structural engendre donc l'arrêt de la production de mélanine.

Ces molécules, du fait de leurs propriétés, ont une affinité pour la protéine transporteuse plus grande que la vitamine A qui sera donc déplacée. Mais l' inventeur a pu constater qu'il ne s'agissait pas seulement de les faire pénétrer dans le mélanocyte, mais aussi d'éviter qu'elles se transforment en vitamine A sous l'influence des enzymes membranaires afin de leur conserver leur plus grande capacité d'antagonisme.

Ces molécules doivent donc être enfermées dans un vecteur qui permettra à la structure chimique d'atteindre le récepteur pour lequel il a une certaine affinité, en tant que pseudo-précurseur, mais non pas d'activité puisqu'il n'est pas le ligand naturel.Ces molécules vont donc pénétrer sansse métaboliser et se comporter alors comme des antivitamines A.

Il importe que le principe actif soit protégé des enzymes membranaires et supporté par une phase particulaire

On a determiné qu'il etait nécessaire d'enfermer le principe actif dans un vecteur liposomique ou nanocapsulaire, c'est-à-dire de taille infra cellulaire.

Les nanosphères sont de petites structures sphériques pleines, dont le diamètre varie de 3 à 1000 nanomètres ; leur taille leur permet de diffuser selon les lois de la diffusion brownienne. Elles peuvent donc être véhiculées par le flux de la solution dans laquelle elles sont dispersées. Ces structures sont en général formulées avec des derivés d'acide polylactique ou polyacrylique ou des mélanges comme ceux communément dénommés : VLDL, constitutifs de la matrice de la nanosphère. Ces nanosphères ont été décrites et etudiées dans quelques ouvrages ou publications de référence, auxquels on se reportera tels que ceux de LEVERGE, DEVISAGAY, PUISIEUX...

Ces nanosphères sont composées de structures métaboliques consommées par les enzymes membranaires.

Il est essentiel que la phase support retenue soit endocytosable par les mélanocytes de l'épiderme, à savoir les cellules claires de Masson et/ou les cellules dendritiques. Pour ce faire, le biologiste mettra en évidence une telle propriété par des essais de routine, par exemple la procédure expérimentale mettant en évidence le déplacement ou l'inhibition d'activité de la dexamethazone ou de l'oestradiol de son récepteur par le catorénoïde ou son homologue vectorisé sur des cultures de cellules épithéliales ou de reins.

L'inventeur a recherche quel pouvait être le vecteur liposomique susceptible de permettre à ces molécules actives de parvenir au niveau cytoplasmique du mélanocyte en passant à travers la membrane cellulaire, sans être transformées par celle-ci.

L'étude des différents types de liposomes lui a permis de découvrir que certains d'entre eux permettaient de transporter une quantité importante de molécules actives lors de la fusion cellulaire : en leur permettant de parvenir au niveau cytoplasmique du mélanocyte, elles vont saturer la protéine transporteuse et donc empêcher les stéroïdes ou la vitamine A d'exprimer leur activité.

Les liposomes M L V (multilamellar large vésicules) ou à grandes vésicules multilamellaires se caractérisent par un empilement important de feuillets phospholipidiques. Ces liposomes sont en général très stables et peuvent encapsuler de grandes quantités d'actifs lipophiles. Toutefois ils ne sont pas adaptes à ce genre de transport parce que l'actif est disperse dans les feuillets. Lors de l'endocytose du liposome par le mélanocyte, seul le premier feuillet externe participera à l'échange moléculaire.

Leur nature lipidique les rendra accessible aux enzymes du métabolisme de l'hélice de Lynen ou aux enzymes lysosomiaux qui dégraderont simultanément les lipides de la membrane et le principe actif transporte. Le principe actif ne sera donc pas libéré au niveau cytoplasmique et ne pourra pas atteindre la protéine transporteuse ni les récepteurs. Elle ne manifestera donc aucun effet.

Les liposomes L U V (large unilamellar vesicules) ou à grandes vésicules unilamellaires ne peuvent encapsuler qu'une petite partie d'actif lipidique car les forces de tension de la paroi ont un effet d'exclusion.

L'inventeur a pu déterminer que les liposomes S U V (small unilamellar vesicules) ou à petites vésicules unilamellaires sont éffiocaces, mais qu'une éfficacité encore supérieure est obtenue par l'utilisation des liposomes renfermant très peu de lamelles ou paucilamellaires,qui sont,eux,les seuls susceptibles de transporter suffisamment d'actif.

Lors de la fusion cellulaire le principe actif sera directement pris en charge à partir de la nouvelle membrane créée par l'échange phospholipidique.

Une importante barrière d'eau obtenue par la fixation de molécules très hydrophiles comme l'acide ascorbique se trouve à l'extérieur des feuillets.

Ce sont ces liposomes, d'envrion 150 à 200 nanomètres, y compris le volume d'hydratation, qui ont été retenus pour constituer les vecteurs selon l'invention.

La présente invention concerne donc l'application de vecteurs liposomiques paucilamellaires, à l'extérieur desquels sont fixées des molécules hydrophiles, au transport à travers la membrane cellulaire de molécules actives exprimant une affinité pour les transporteurs cytoplasmiques.

Cette construction est indispensable pour internaliser avec efficacité, dans la cellule, le principe actif.

Ce volume d'hydratation empêche le relargage du produit actif à l'extérieur de la cellule lors de la fusion ou de l'endocytose.

Selon un mode de réalisation de l'invention, ces vecteurs paucilamellaires, de structure fusionnante avec les cellules sont aptes à conduire, à travers la membrane cellulaire des molécules actives susceptibles de se fixer sur les protéines cytoplasmiques transporteuses des stéroïdes.

Selon un autre mode de réalisation, les vecteurs paucilamellaires sont des vecteurs de structure fusionnante avec les membranes des cellules susceptibles d'amener un antagoniste de la vitamine A apte à se fixer sur la protéine cytoplasmique transporteuse de la vitamine A.

La molécule active peut manifester un blocage de la formation des mélanosomes par detournement de l'activité enzymatique se traduisant par une hyperactivation des enzymes de dégradation des mélanines.

La molécule active peut rendre les cellules insensibles aux stimuli de l'activation mélanocytaire se traduisant par une absence d'activation des synthétases.

La molécule active est choisie parmi les molécules qui présentent une affinité pour les récepteurs ou les transporteurs de la vitamine A et des stéroïdes et donc une certaine analogie de structure avec ceux-ci.

Selon l'invention, les activateurs de la fonction mélanoblastique sont avantageusement choisis parmi les homologues structuraux partiels de la vitamine A non oxydés ou peu oxydés.

Ces homologues structuraux partiels de la vitamine A non oxydes ou peu oxydes sont choisis de préference parmi le β-carotène, l'apo-8-caroténal, le farnésol, le lycopène, le phytol, le phytofluène et le β-citraurine

Les inhibiteurs de la synthèse mélanoblastique sont choisis parmi les homologues structuraux non précurseurs de la vitamine A, oxydés sous forme alcoolique ou cétonique,et de préference parmi les caroténoïdes comme l'astaxanthine, l'astacine,la canthaxantine, la zéaxanthine, la chrysanthémaxantine.

Selon l'invention, les molécules actives sont également choisies parmi les molécules susceptibles de déplacer la vitamine A de son transporteur ou de son récepteur, comme certains flavonoïdes et certaines vitamines comme la vitamine K et la vitamine E.

Enfin les vecteurs paucilamellaires selon l'invention présentent un potentiel zéta de -30 à -80 mV. On empêche ainsi la vésicule de se fixer sur les érythrocytes et on les mobilise au niveau des assises épidermiques afin de permettre une fusion avec les mélanocytes.

Les exemples suivants permettront de mieux comprendre l'invention, sans aucunement la limiter.

**Exemple 1 -**

$10^6$ de fibroblastes cultivées en milieu M E M modifié sont traitées par une solution $10^{-7}$ M d'oestradiol. On observe une puissante constriction des cytoplasmes.

Les cellules restent vivantes mais ne retrouvent pas leur aspect fusiforme même après rinçage du milieu. Par contre si, dans le liquide de rinçage, on ajoute un peu de sérum de liposome paucilamellaire contenant 300 ppm de β-carotène ou apo-8-caroténal de telle manière que la quantité globale de lipides soit d'environ 50 μg / ml de solution nutritive, on observe que les fibroblastes retrouvent un phénotype normal ; leur cytoplasme s'hyperactive et devient granuleux.

Les cellules cultivées en présence de 500 ppm d'astaxanthine, de zéaxanthine, d'astacine ou de canthaxanthine, encapsulé dans des liposomes paucilamellaires de 150 nm (la dose de lipide total dans le liquide nutritif n'étant pas supérieure à 100 μg /ml) puis traitées par $10^{-7}$ M d'oestradiol ne voient pas leur phénotype changé. Elles conservent un cytoplasme clair et un aspect fusiforme.

Les cellules traitées par l'oestradiol à $10^{-7}$ M puis rincées par un milieu nutritif à 100 μg / ml de lipides contenu dans des vésicules liposomiales à 500 ppm d'astaxanthine, zéaxanthine, astacine ou canthaxatine retrouvent un phénotype normal.

**Exemple 2**

Des fibroblastes sont cultivées en présence d'une solution $10^{-7}$ M d'oestradiol puis rincées par une solution MEM contenant 100 μg /ml de phospholipide encapsulant 300 ppm de β-carotène ou d'apo-8-caroténal ; elles présentent, au bout de 4 h un taux d'oestradiol dans les surnageants de 32 μg alors que les cellules rincées sans liposomes ont une teneur d'oestradiol de 52 μg.

Les mêmes fibroblastes rincées par un sérum liposomial à l'astaxanthine, l'astacine, la zéaxanthine et la canthaxanthine ont un taux d'oestradiol de 63 μg.

On note que l'aspect granuleux des cytoplasmes correspond à une activation des fibroblastes : activation objectivée par une dégradation rapide des stéroïdes. Il y a détournement de l'activité de la cellule.

Le mélanocyte n'est plus sensible à l'action des stéroïdes et ne développe pas de systèmes transporteurs susceptibles d'augmenter la mélanogenèse.

Avec un effet légèrement différent, les actifs homologues structurels mais non précurseurs de la vitamine A rendent la cellule insensible au stress cellulaire et empêche la mélanogenèse par occupation des sites transporteurs des signaux activateurs de la synthèse de la mélamine.

Les molécules peuvent donc pénétrer sans se métaboliser ; elles vont alors se comporter comme des anti-vitamines A. Le choix du vecteur liposomique est important car il doit avoir une forte affinité pour le mélanocyte. La membrane de la nanocapsule va fusionner avec les structures membranaires ce qui permettra aux molécules de passer au niveau cytoplasmique sans être metabolisées en vitamine A ou en ligands ayant perdu leur affinité pour le transporteur cytoplasmique.

On obtient alors une dépigmentation par mise en sommeil provisoire du processus normal de formation de la mélanine. Il n'y a donc pas d'altération irreversible d'un mécanisme dont le déroulement physiologique doit toujours être préservé.

Par ailleurs, les propriétés anti radicaux libres de ces substances peuvent être avantageusement mises à profit pour protéger la peau contre l'action des radiations solaires.

L'invention concerne donc également les compositions dermatologiques ou cosmétiques à base de vecteurs liposomiques selon l'invention qui sont utilisée pour le traitement de dyschromies telles que les éphélides non photoinduites, les masques gravidiques, les leucodermies, les photodermies en breloque d'origine chimique, les hyperpigmentations d'origine hormonale ou traumatique. Le principe actif selon l'invention agit par effet blanchissant en bloquant la production de la mélanine, son effet inhibiteur intervenant au niveau du complexe qu'il forme avec les récepteurs de la vitamine A et des stéroïdes ou de leurs transporteurs.

Les compositions selon l'invention peuvent également traiter les anomalies liées à l'exposition solaire, en retardant la formation de mélanomes.

Les exemples suivants de formulations illustreront bien l'invention sans nullement en limiter la portée.

EXEMPLE 3; Serum 1

– Astacine d'origine micro-organique en solution chloroformique à 10 % : 0,500 g
– Lécithine de soja : 10,0 g
– cholestérol en solution chloroformique à 15 % : 3,805 g
– Ligand de surface : 0,050 g
– DL-beta et gamma-tocophérol : 0,050 g

Les vésicules sont formées après évaporation du chloroforme sous une grande agitation tourbillonnaire à 300 mbar.

Le Serum 1 obtenu est utilise pour formuler des gels ou des crêmes.

Exemple de formulation d'un gel à partir du serum 1 :
– Carbomère (Carbopol 934 P) : 500,0 mg
– Serum 1 : 4,50 mg
– Solution d'hydroxyde de sodium 10 % q.s.p. 7,300 mg

EXEMPLE 4 : Sérum 2

- Phytol : 0,400 mg
- Lécithine d'oeuf : 8,250 g
- Sitostérol : 4,30 g
- DL béta et gamma-tocophérol : 0,005 g
- Eau qsp 100,0 g

Les capsules sont formées après passage sur la presse de FRENCH à 20000 psi (1.379 bars), selon la méthode de Barenholzt, Anselm et D. uchtenberg ou mieux selon celle de R.L. Mamilton et coll. (1980)

Le sérum 2 obtenu est utilise pour formuler des gels ou des crêmes.

Exemple de formulation d'une crème à partir du sérum 2:
- Apifil : 80 ,0 g
- alcool cetylique :10,0 g
- isostéarate d'isostéaryle : 170,0 g
- sérum 1 : 50,0 g
- huile de vaseline : 48,0 g
- palmitate d'ascorbyle : 5,0 g
- eau : 625,0 g
- Carbopol : 940 : 3,0 g
- triéthanolamine :(sol à 50 %) : 6,0 g
- parfum : 3,0 g

EXEMPLE 5 : Formulation d'une composition à base de β-carotène appliquée à la dépigmentation

Huit patients, cinq femmes et trois hommes, âges de 41 à 80 ans, ont accepte de tester le β-carotène inclu dans des liposomes paucilamellaires pour étudier son effet dépigmentant. L'un était de phototype II,un autre de phototype IV, les six autres de phototype III. Tous présentaient une dépigmentation photo-induite du décolleté, du dos des mains et des avant-bras. L'ancienneté de ces taches pigmentaires remontait à plus de dix ans.

Le produit a été appliqué chez les huit patients sur une zone pigmentée découverte, en prenant comme référence la zone symétrique non traitée. Sept patients sur huit ont effectué un traitement identique sur des taches pigmentées en zone de peau couverte. L'application a été éffectuée matin et soir pendant 28 jours sur un seul côté du corps, toujours le même. Les résultats obtenus sont les suivants :

peau exposée : six résultats positifs sur huit
peau couverte : six résultats positifs sur sept;

Dans tous les cas positifs, une décoloration a été notée par le patient et le médecin. Cette dépigmentation intéresse non seulement les taches photo-induites, mais l'ensemble du tégument. Bien que les patients n'aient utilisé le produit que pendant un mois, la dépigmentation est incontestable même si le plus souvent elle reste minime.

On peut donc penser qu'en période hivernale, un traitement semblable, conduit pendant quatre à six mois, puisse permettre d'obtenir de véritables décolorations des pigmentations photo-induites.

Résultats : un seul patient n'a pas eu de zone couverte exploitable. Chez tous les autres, on observe :
- en zone exposée : 6 améliorations , le plus souvent discrètes, sur 8, avec parfois majoration de la pigmentation de la zone témoin du fait de l'ensoleillement.
- en zone couverte : 6 améliorations sur les 7 étudiées, ce qui autorise à proposer comme dit plus haut,un test de ce type en periode hivernale.

D'une manière générale, on a toujours observé une augmentation du fond jaune du tégument.

Exemple 6 -

Des essais ont été éffectués, dans les mêmes conditions que dans l'exemple 5, en remplaçant le β-carotène par l'astoxanthine.

Les résultats observés sont les suivants :
- en fonction de la zone :
  - 5 tests sur 7 sont positifs, tant en zone couverte qu'en zone découverte, soit 71,4 % .
- en fonction de la carnation de la peau :
  - pour les peaux mates, 9 tests sur 10 s'avèrent positifs, soit 90 % de réussite ;
  - pour les peaux claires, 2 tests sur 4 sont positifs, soit 50 % de réussite.
- en fonction de la lésion :
  - 9 cas sur 12 sont positifs sur les pigmentations photo-induites, soit 75 % dont 80 % de réussite en zone couverte et 67 % en zone découverte.
  - 1 cas positif sur 1 s'observe sur une keratose actinique, en zone couverte.

Dans tous les cas, on observe une diminution du fond jaune du tégument.

**Revendications**

**1** - Application de vecteurs liposomiques paucilamellaires, à l'extérieur desquels sont fixées des molécules hydrophiles, au transport, à travers la membrane cellulaire, de molécules actives exprimant une affinité pour les transporteurs cytoplasmiques.

**2** - Vecteurs paucilamellaires selon la revendication 1, caractérisés en ce que ces vecteurs, de structure fusionnante avec les cellules, sont aptes à conduire, à travers la membrane cellulaire des molécules actives susceptibles de se fixer sur les protéines cytoplasmiques transporteuses des stéroïdes.

**3** - Vecteurs paucilamellaires selon la revendication 1 et la revendication 2, caractérisés en ce qu'ils transportent des molécules actives choisies parmi les molécules qui présentent une affinité pour les récepteurs ou les transporteurs de la vitamine A et des stéroïdes et donc une certaine analogie de structure avec ceux-ci.

4 - Vecteurs paucilamellaires selon la revendication 3, caractérisés en ce que les molécules actives sont des activateurs de la fonction mélanoblastique, choisis parmi les homologues structuraux partiels de la vitamine A non oxydés ou peu oxydés.

5 - Vecteurs paucilamellaires selon la revendication 4, caractérisés en ce que les homologues structuraux partiels de la vitamine A non oxydés ou peu oxydés sont choisis parmi leβ-carotène, l'apo-8-caroténal, le farnésol, le lycopène, le phytol, le phytofluène et le β-citraurine

6 - Vecteurs paucilamellaires selon la revendication 1, caractérisée en ce que ces vecteurs, de structure fusionnante avec les membranes des cellules, sont susceptibles d'amener un antagoniste de la vitamine A apte à se fixer sur la protéine cytoplasmique transporteuse de la vitamine A.

7 - Vecteurs paucilamellaires selon la revendication 2 et la revendication 6, caractérisés en ce qu'ils transportent des molécules actives choisies parmi les molécules qui manifestent un blocage de la formation des mélanosomes par détournement de l'activité enzymatique se traduisant par une hyperactivation des enzymes de dégradation des mélanines.

8 - Vecteurs paucilamellaires selon la revendication 6 et la revendication 7, caractérisés en ce que les inhibiteurs de la synthèse mélanoblastique sont choisis parmi les homologues structuraux non précurseurs de la vitamine A, oxydés sous forme alcoolique ou cétonique.

9 - Vecteurs paucilamellaires selon la revendication 8, caractérisés en ce que les homologues structuraux non précurseurs de la vitamine A sont choisis parmi les caroténoïdes comme l'astaxanthine, l'astacine, la canthaxantine, la zéaxanthine, la chrysanthémaxantine.

10- Vecteurs paucilamellaires selon la revendication 6 et la revendication 7, caractérisés en ce que les molécules actives sont choisies parmi les molécules susceptibles de déplacer la vitamine A de son transporteur ou de son récepteur.

11 - Vecteurs paucilamellaires selon la revendication 10, caractérisés en ce que les molécules actives sont choisies parmi les molécules susceptibles de déplacer la vitamine A de son transporteur ou de son récepteur.

12 - Vecteurs paucilamellaires selon la revendication 11, caractérisés en ce que les molécules actives sont choisies comme les flavonoïdes et certaines vitamines comme la vitamine K et la vitamine E.

13 - Vecteurs paucilamellaires selon l'une des revendications 1 à 12, caractérisés en ce qu'ils transportent des molécules actives choisies parmi les molécules qui rendent les cellules insensibles aux stimuli de l'activation mélanocytaire.

14 - Vecteurs paucilamellaires selon l'une des revendications 1 à 12 caractérisés en ce qu'ils présentent un potentiel zéta de -30 à -80 mV.

15 - Compositions dermatologiques ou cosmétiques caractérisées en ce qu'elles sont essentiellemùent constituées d'un vecteur paucilamellaire selon l'une quelconque des revendications 1 à 14.

16 - Applications des compositions dermatologiques ou cosmétiques selon la revendication 15 à la supression ou à l'atténuation de dischromies.

17 - Applications des compositions dermatologiques ou cosmétiques selon la revendication 15 au traitement des mélanomes engendrés par l'exposition solaire.